# EUROPEAN PATENT APPLICATION

(11) **EP 1 428 872 A2**
(43) Date of publication of application: **16.06.2004**
(21) Application number: 04004512.2
(22) Date of filing: 12.04.2001
(51) Int. Cl.: C12N 5/06, A61K 35/26, A61K 35/28, A61K 35/23, A61K 35/407

(54) **Tolerance-inducing thymus-bone marrow composite tissue construct and organ**

(30) Priority: 17.04.2000 US 550144
(62) Divisional of application: 01938113.6
(71) Applicant: Novartis AG, 4056 Basel (CH); Novartis Pharma GmbH, 1235 Vienna (AT)
(72) Inventor: Lake, Philip, Morris Plains NJ 07950 (US); Shi, Victor Chengwei, Short Hills NJ 07078 (US)
(74) Representative: Grubb, Philip William

(57) **Abstract**

Thy-marrow composite tissue construct and composite organ for creating hematopoietic chimersim and inducing long-term stable immunological tolerance in a transplant recipient, especially a human xenotransplant recipient of a non-human mammalian (e.g. pig) organ.

## Description

### Field of the Invention

The present invention provides a composite tissue implant, a mammalian organ comprising said composite tissue implant for use in organ transplantation, a non-human mammal comprising said organ, and methods for achieving hematopoietic chimerism and stable donor-specific immunological tolerance in a mammalian transplant recipient.

### Background of the Invention

The induction of donor-specific immunological tolerance remains an elusive goal in allo- and xeno-transplantation research. The term "immunological tolerance" refers to a state of unresponsiveness by the immune system of a patient subject to challenge with the antigen to which tolerance has been induced. In the transplant setting, in particular, it refers to the inhibition of the graft recipient's ability to mount an immune response which would otherwise occur in response to the introduction of non-self MHC antigen of the graft into the recipient.

Recent studies have focused on thymic grafts as means of developing immunological tolerance in the transplant recipient (i.e. "host"). For example, Nikolic and co-workers showed that porcine thymus grafts in immunodeficient mice supported normal development of polyclonal, functional human T cells; and that the T cells were specifically tolerant to donor-specific MHC Ags, Journal of Immunology, 1999, 162:3402-3407 (1999). Zhao, and co-workers reported that grafting of transiently immunosuppressed mice with fetal pig thymus and liver conferred tolerance in the murine recipient to a subsequent porcine skin graft, Nature Medicine 2:1211-1216 (1996).

Lambrigts and co-workers introduced the concept of a "thymo-organ" comprising autologous thymic grafts under the kidney capsule or in the heart of a swine model [Lambrigts et al., Xenotransplantation 3:296- (1996)]; and reported obtaining stable intracardiac engraftment of the thymic tissue in a heart later transplanted [Larnbrigts et al., Transplantation 66:810- (1998)]. Yamada and co workers report that in a miniature swine model, thymectorized class I-disparate recipients of a composite allogeneic "thymokidney" (kidney with vascularized autologous thymic tissue under its capsule) receiving a 12-day course of cyclosporine, had stable renal function with no evidence of rejection, and donor-specific unresponsiveness. By postoperative day ("POD") 14, the thymic tissue in the thymokidney contained recipient-type dendritic cells; and by POD 60, recipient-type class I positive thymocytes appeared in the thymic medulla, indicating thymopoiesis. T cells were found to be both recipient and donor MHC-restricted. It was concluded by Yamada and co-workers that the presence of vascularized donor thymic tissue is capable of inducing tolerance to class I-disparate kidney allografts in thymectomized recipients [Yamada et aL, Journal of Immunology 164:3079-3086 (2000); Yamada et al., Transplantation 68:1684-1692 (1999); see also Sachs, Clinical Immunology 95:S63-S68 (2000)].

However, we believe that the thymo-organ as previously described is inadequate for maintaining long-term, stable immunological tolerance in either an allogeneic or xenogeneic setting. In particular, one of the essential functions of the implanted thymic tissue is to provide a site for negative selection of developing thymocytes for self-tolerance-by dendritic cells. In the heretofore disclosed thymo organ model, this function is eventually compromised by the irreversible depletion of donor-derived dendritic cells of the graft tissue. Dendritic cells in general have a half life of approximately one month or less. Thus, over time the capacity for negative selection of developing thymocytes to maintain tolerance to the donor will be lost.

Currently, there are no reported means of maintaining production and availability of dendritic cells or their precursors to a thymo-organ. Thus the apparent tolerance achieved by prior workers in the field must be only transient at best.

### Summary of the Invention

We have now devised a novel composition and means for achieving hematopoietic chimerism in a graft recipient, and for inducing stable, long-term tolerance in a mammalian recipient of an allogeneic or xenogeneic transplanted organ. More specifically, our invention contemplates implanting in the organ recipient a novel, self-replenishing source of donor (or donor-histocompatible) hematopoietic cells, including dendritic cells or their precursors. This novel source comprises the substantially intact hernatopoietic stromal micoenvironment (HSM) of the bone marrow. An example of such a substantially intact HSM comprises an intact bone marrow plug or cylinder, preferably on the order of 1-5 mm3, which has been surgically removed from the bone marrow of a mammalian donor. Unlike typical injectable bone marrow preparations, which consist essentially of a stem cell inoculum in an otherwise non-functional detritus of disrupted tissues and cells, the novel, substantially intact HSM component of the invention provides a mico-environment which is favorable for continued hematopoiesis within the thymo-organ graft.

In particular, it is our discovery that a composite tissue construct comprising as a first component, donor (or donor-histocompatible) thymic tissue and as a second component, substantially intact tissue of the hematopoietic stromal microenvironment (HSM) of donor (or donor- histocompatible) bone marrow, provides de novo and continuous production of donor-type dendritic cells in the transplant recipient; and furthermore, that such a vascularized composite tissue construct provides a sustained, self-replenishing source of donor-type dendritic and other bone marrow-derived cells capable of migrating to the donor thymic tissue and assisting in thymocyte differentiation and development.

In a preferred embodiment of the invention, a "thymo-bone marrow" - alternately referred to herein as a "thy-marrow" -composite organ" is prepared by implanting the just described composite tissue construct as a unitary device in the donor organ.

In an alternative embodiment of the invention, a thy-marrow-compo site organ may be prepared by separately implanting the above-described thymic tissue component and bone marrow HSM component in the organ.

T-cell-depleted or otherwise conditioned recipients of such a thy-marrow composite organ will become tolerized to the organ, because the regenerating T cell population of the host will have matured (via positive and negative selection) in the presence of sustained production of marrow-derived dendritic cells of the donor.

The resulting mixed chimeras are both immunocompetent and tolerant to self as well as to donor. Long term tolerance is possible because the invention provides for continued replenishment of dendritic cells, insuring the continued functionality of the thymic or peripheral negative selection or anergic process within the recipient, and thus a self-perpetuating state of donor-specific tolerance to the graft. Our invention provides a means of securing long-term, stable tolerance to transplanted tissues or organs, in the absence of rejection or aberrant auto-immune responses, by providing essentially a tranplantable, portable "engine" of tolerance in the composite tissue construct of the invention.

The thy-marrow composite tissue construct and composite organ of the invention may be utilized for inducing tolerance in a mammalian transplant recipient of an allogeneic (ie. of the same species) or xenogeneic (i.e. of a different species) organ. The composite tissue construct and organ of the invention are particularly useful for creating hematopoietic chimerism and inducing tolerance in a xenotransplant recipient, especially a human recipient of a non-human mammalian (especially, Sus scrofa i.e. swine) organ.

### Brief Description of the Drawings

FIG. I Schematic view of an embodiment of a "portable engine" for tolerance induction according to the invention. The depicted composite tissue construct comprises mingled thymus and bone marrow tissue sections within a supporting means derived from a section of intestinal sac (see left panel). Also schematically depicted (right panel) is the network of blood vessels connecting the vascularized composite with the surrounding tissue of its mammalian host.
FIG. 2 Schematic depiction of a composite "bone marrow-kidney", a "thymokidney", and a "thymo-bone marrow (a.k.a. 'thy-marrow')-kidney" according to the invention.

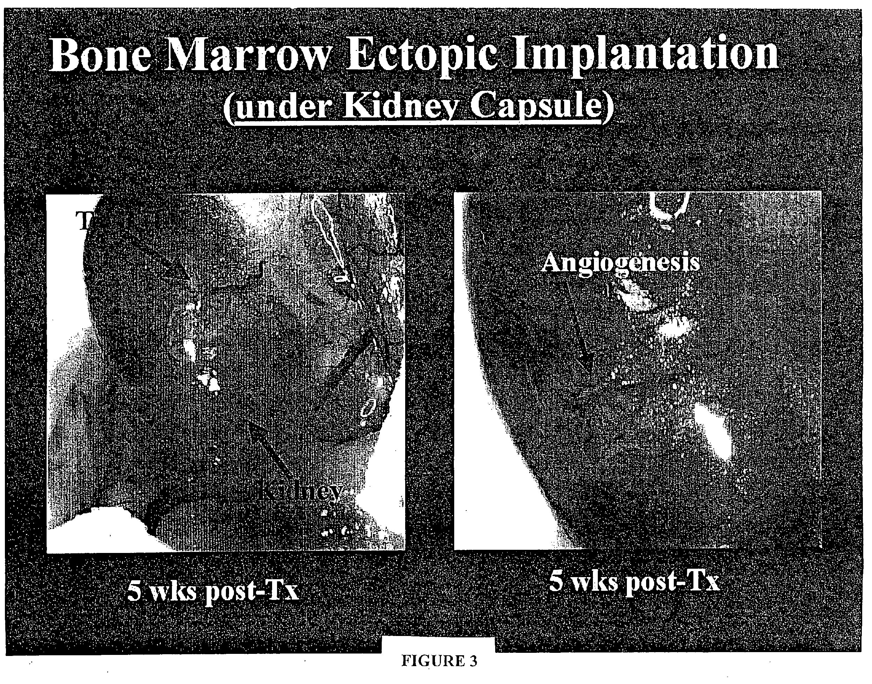

FIG. 3 Macroscopic appearance of mouse kidney capsule containing ectopically implanted syngeneic bone marrow stroma (left panel) or bone marrow implanted under kidney capsule (right panel) (arrow indicates bone marrow stroma and kidney). At 5 weeks post-transplantation ("Tx"), extensive angiogenesis has been formed around BM implanting site, the bone marrow appearing as white tissue.

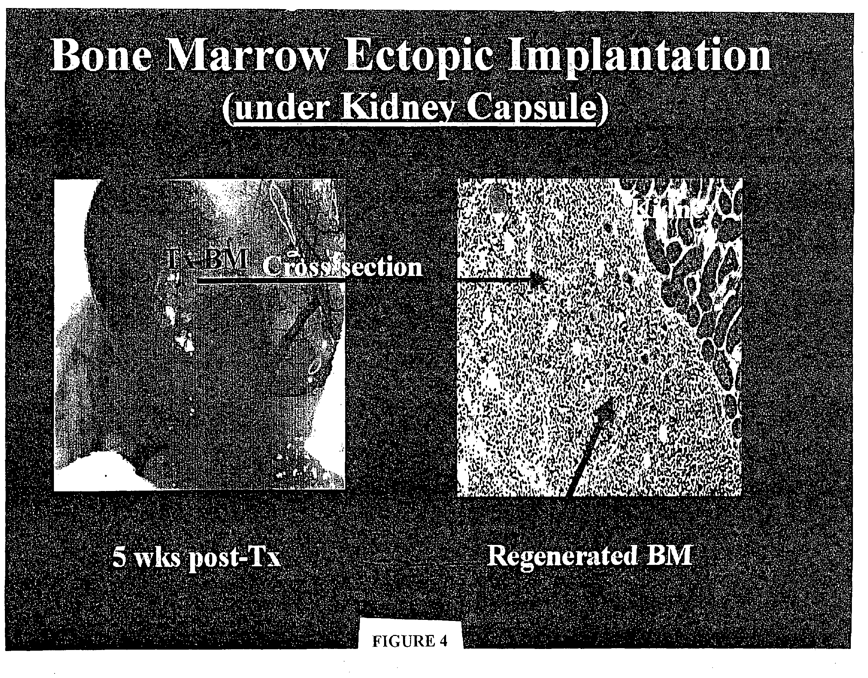

FIG. 4 (left panel) Additional macroscopic image of mouse kidney capsule as described in connection with Fig. 3. (right panel) Photomicrograph of cross- section of bone marrow stromal implant at 5 weeks post-transplant showing "regenerated", i.e. blood-suffused, bone marrow (20x, H & E staining).

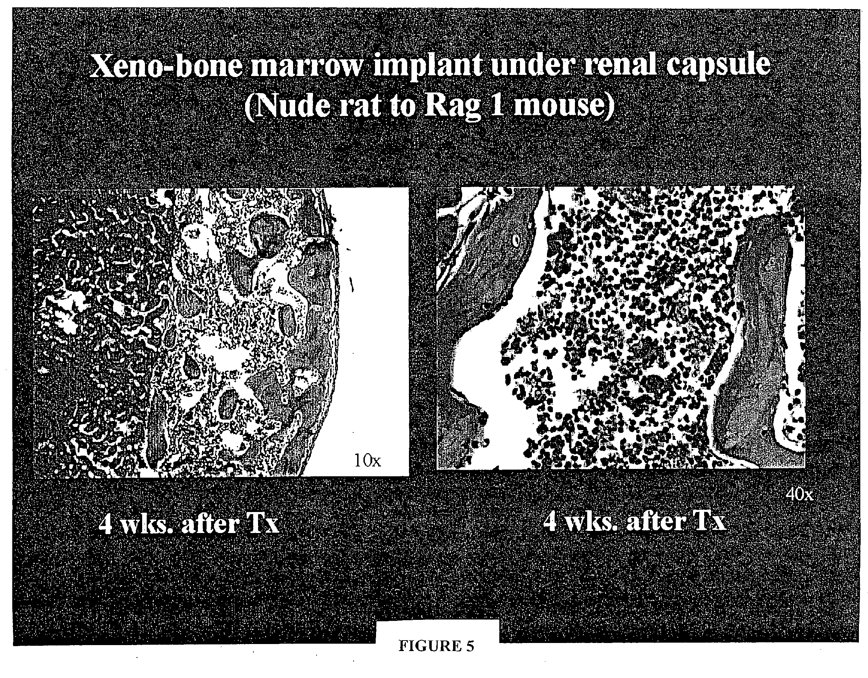

FIG. 5 10x (left panel) and 40x (right panel) photomicrographs of section of xenogeneic (nude rat to RAG I mouse) bone marrow ectopically implanted under mouse kidney capsule, 4 weeks after transplant (H & E staining).

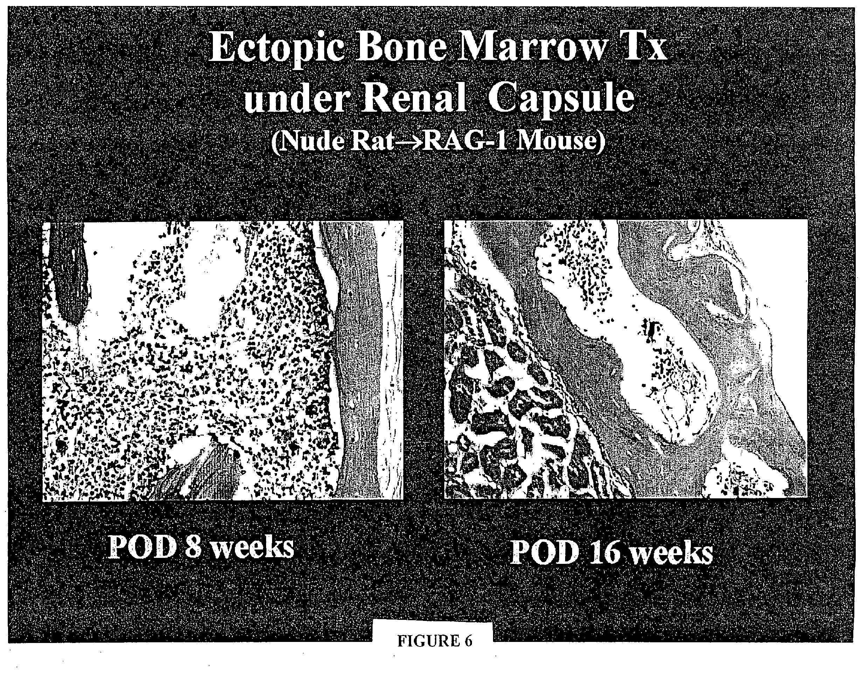

FIG. 6 20x photomicrographs of section of xenogeneic (nude rat to RAG- I mouse) bone marrow ectopically implanted under mouse renal capsule at 8 weeks (left panel) and 16 weeks (right panel) post-Tx (H & E staining).

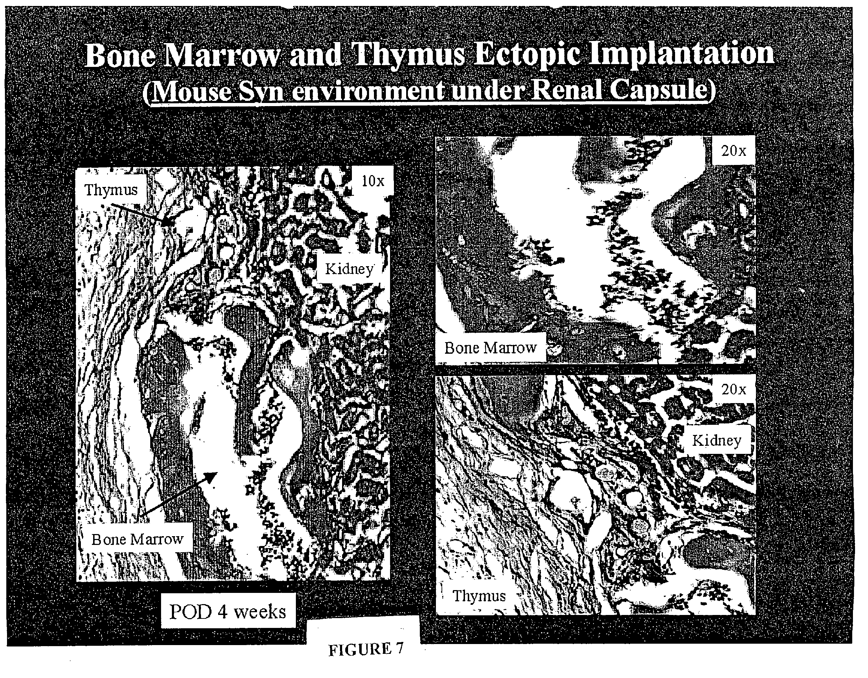

FIG. 7 10x (left panel) and 20x (right panel) photomicrographs of syngeneic bone marrow and thymus tissue ectopically implanted under kidney capsule in (H & E staining) mouse model at 4 weeks post-Tx (H & E staining).
FIG. 8 Photomicrographs of H&E-stained section (left panel) and immunohistochemical stained section (for CD 45 positive-leukocytes) (right panel) of xenogeneic (rat to mouse) bone marrow ectopically implanted under kidney capsule. Arrows point to the bone marrow implant (left panel) and to CD45+ leukocytes in bone marrow implant (right panel).
FIG. 9 Photomicrographs of mouse anti-rat CD45-stained (left panel) and rat antimouse CD45-stained (right panel) cross-sections of mouse spleen to confirm the absence of cross-reactivity.

### Detailed Description of the Invention

The tolerance-inducing composite tissue construct of the invention comprises functional thymic tissue and substantially intact hematopoietic stromal microenvironment (HSM) of the bone marrow.

It is essential that the thymic tissue component of the composite tissue construct of the invention supply a sequestered and architecturally organized microenvironment which is conducive to development of mature T cells from naive thymocytes.

### T-cell maturation in the thymus.

The process of T-lymphocyte maturation through the thymocyte stage, to form mature T-cells, has been well- characterized [see, for example, Charles A. Janeway and Paul Travers, Immunobiology - the Immune System in Health and Disease, 2d ed., Ch. 6, Current Biology Ltd./Garland Publishing Inc. (1996)]. Briefly,T-lymphocytes by differentiation of lymphoid precursors in the hematopoietic stroma of the bone marrow migrate from the bone marrow to the periphery and eventually reach the thymus, where the cells are subjected to various steps of differentiation and selection, primarily in the cortex (the medulla generally containing only mature T-cells). The lymphocytes first enter the thymus as double- negative cells, expressing neither the T-cell receptor nor either of the two co-receptor molecules, CD4 and CD8, and become embedded in an epithelial network known as the thymic stroma, which provides an inductive microenvironment for differentiation and development. Following an initial phase of proliferation in the subcapsular zone of the cortex, the lymphocytes (also referred to as "subcortical lymphoblasts") differentiate into double-positive cells, expressing the T-cell receptor and both CD4 and CD8 co-receptors. The resulting, immature thymocytes ("cortical thymocytes") thereafter undergo two types of selection: positive selection for recognition of self MHC:self peptide complexes, and negative selection for recognition of self peptide; self NMC complexes that would otherwise trigger the T cell in the periphery.

### Positive Selection of Thymocytes.

Positive selection of thymocytes is normally mediated in the cortex by MIFIC-bearing cortical epithelial cells. Positive selection ensures that all mature T cells will be able to respond to foreign peptides presented by self-NMC, i.e. that the mature T cells will be "self NUIC-restricted". Cells that do not encounter their restricting MHC molecules on the thymic epithelium remain double positive and die in the thymus within 3 or 4 days of their last division, to be scavenged by macrophages.

Thymocytes that survive the positive selection process express only one of the two co-receptors, CD4 or CD8. Additionally, mature CD4-bearing T-cells have receptors that recognize peptides bound to self MHC class II molecules, whereas the CD8-bearing T-cells have receptors that recognize peptides bound to self MHC class I molecules. Thus, positive selection also determines the cell surface phenotype of the mature T cell, equipping it with a co-receptor that it requires for efficient antigen recognition.

### Negative Selection of Thymocytes.

The double-positive cells must also undergo a purging process in which potentially self-reactive T cells are eliminated. The purging function of the thymus is referred to as negative selection. Negative selection is thought to be most stringent at the corticomedullary junction, where nearly mature, "medullary thymocytes" encounter antigen-presenting cells (APQ that also activate mature T cells in the peripheral lymphoid tissues. The APC are comprised of bone marrow- derived dendritic ("interdigitating") cells, as well as macrophages. The self antigens presented by these cells are, therefore, the most important source of potential autoimmune responses, and T cells responding to such self peptides must be eliminated in the thymus. Clonal deletion by the self peptide: self MHC complex generates a repertoire of mature T cells that does not respond to the self-antigens expressed by its own APC, establishing self-tolerance. The surviving mature T-cells exit from the medulla to the peripheral circulation [see Janeway and Travers, id. at 6:15-6:31]. Thus, both positive and negative selection are necessary concomitants for producing a useful and non-damaging repertoire of T-cell receptors.

Accordingly, by "functional thymic tissue" is meant tissue of the thymus having a microenvironment which is capable of processing naive thymocytes to the mature Tcell stage in a mammal. Therefore the thymic component of the composite tissue structure, as well as of the composite organ of the present invention, in order to constitute "functional thymic tissue" should, in particular, comprise a sufficient portion of thymic stroma, including cortical epithelial cells and medullary epithelial cells, to support positive and negative selection of thymocytes to yield mature T cells having donor self- recognition and donor self-tolerance.

Since the thymus tends to shrink or involute after puberty, in practice it may be difficult if not impossible to derive sufficient or functioning autologous thymic tissue from an adult organ donor. Accordingly, in one embodiment of the invention, the thymic tissue is derived from an animal which is histocompatible to the organ donor. An example of a histocompatible animal is a mammal of the same inbred strain as the organ donor. Thus thymic tissue may be harvested from a juvenile (or alternatively, neonatal or fetal) donor swine and implanted into an organ of an adult swine organ donor of identical inbred strain.

The term "implant" or "transplant" or "graft" as used herein shall be understood to refer to the act of inserting tissue or an organ into a living mammalian recipient under conditions that allow the tissue or organ to become vascularized; and shall also refer to the so-inserted (i. e. "implanted" or "transplanted" or "grafted") tissue or organ. Conditions favoring vascularization of a graft in a mammal comprise a localized tissue bed at the site of the graft having an extensive blood supply network. For example, an appropriate site in the kidney for insertion of tissue or an organ to promote vascularization is under the renal capsule, adjacent to the parenchyma. Appropriate sites in the heart in which to insert tissue or an organ to promote vascularization comprise the subepicardial fat pads overlying the right and left atrioventricular grooves; the right and left atrial appendages; the aortopulmonary window; or the free wall of the right ventricle.

Surgical removal of the thymus or part thereof from the thymus donor should be carried out with care to preserve the integrity of the thymus stromal microenvironment. The thymic tissue may be removed from its native environment by careful dissection.

The second component of the composite tissue construct of the invention comprises a functional hematopoietic stromal microenvironment (HSM) of the bone marrow. The HSM should be histocompatible with the thymic tissue component, as well as with the organ donor.

By "functional hematopoietic stromal. microenvironment" is meant tissue obtained from bone marrow having a microenvironment which is capable of effecting hematopoietic development and differentiation in a mammal.

### Hematopoietic Stromal Microenvironment

The process by which T lymphocytes arise in the bone marrow as a result of the differentiation of lymphoid progenitor cells descended from totipotent hematopoietic stem cells, has been intensively studied.

### The stromal

Cells include endothelial cells that form the sinuses of the bone marrow and adventitial reticular cells that have characteristics consistent with adipocytes, fibroblasts, and muscle cells [Chabord et al., Blood 66:1138 (1985), Chabord et al., Exp. Hematol. 18:276 (1990)]. It has long been appreciated that stromal cells in the marrow provide the structural scaffolding for hematopoiesis [Mayani et al. Eur. J. Hatmat. 49:225-233 (1992)]. Certain studies have shown that direct stromal. cell-to-blood cell contact, stromal cell production of the extracellular bone marrow matrix, and cytokine synthesis by stromal cells are all relevant to the formation of various blood cells [see U.S. Patent No. 5,733,541, incorporated by reference]. Direct contact with the stromal cells has been found necessary for stem cell maintenance in long term bone marrow culture [Dexter et al., Ann. Rev. Cell. Biol. 3:432-441 (1987)]. Human marrow stromal cell lines have been established which sustain hematopoiesis [see, e.g., U.S. Patent No. 5,879, 940, incorporated by reference]. It has been reported that stromal cells are capable of transferring the hematopoietic microenvironment of the donor after allogeneic bone marrow transplantation. Gurevitch and co-workers reported data suggesting that transplantation of donor bone marrow within the hematopoietic stromal microenvironment may increase donor cell chimerism and provide conditions of long term survival of both allogeneic and xenogeneic grafts, Transplantation 68:1362-1368 (1999).

The hematopoietic stromal component of the thy-marrow composite tissue construct and organ of the present invention, must provide a sufficiently intact microenvironment so that hematopoietic development is maintained once the tissue is revascularized in the recipient.

Accordingly, surgical removal of bone marrow may be carried out using well known surgical techniques to preserve the integrity of the bone marrow hematopoietic stromal microenvironment. Suitable sources of marrow include the long bones (femora) and the tibia. For example, marrow may be mechanically pressed out of the femoral canal by a mandrin or trocar to yield plugs in which the HSM is left substantially intact.

Alternatively, cultured hematopoietic stromal cell cultures, as described by the literature (see, e.g., U.S. Patent No. 5879,940, incorporated by reference) may be utilized to provide the required hematopoietic stromal microenvironment of the invention.

Preferably, the thymic tissue component and the bone marrow component are each irradiated to reduce the number of hematopoietic progenitors. Irradiation may be carried out in vivo i.e. prior to removal (in the case of a non-human mammalian donor) and/or ex vivo, i.e. following removal and prior to implanting in a recipient, 5 and/or after re-implanting in a non-human recipient.

The bone marrow may be harvested from either the donor mammal providing an organ for transplantation or from a histocompatible individual (e.g., of the same inbred strain).

To prepare a composite tissue construct or organ of the invention, it is essential that the thymic tissue and bone marrow tissue be arranged within the tissue construct or organ in intimate association.

By "intimate association" is meant that the two types of tissue are sufficiently proximately disposed that hematopoietic cells (e.g. dendritic cells or their precursors) released from the bone marrow tissue are capable of migrating to the thymic tissue.

The intimate association, i.e. close physical positioning, of the two types of tissue is an essential factor in achieving tolerance induction, since such intimate association is needed for efficient transiting of dendritic cell precursors from the HSM bone marrow to the thymic tissue, where the dendritic: cells play a key role in deletional selection of thymocytes to maintain graft self-tolerance. Said migration may be through tissue parenchyma, following chemotactic, gradients, or by entry into the blood circulation and/or lymphatics.

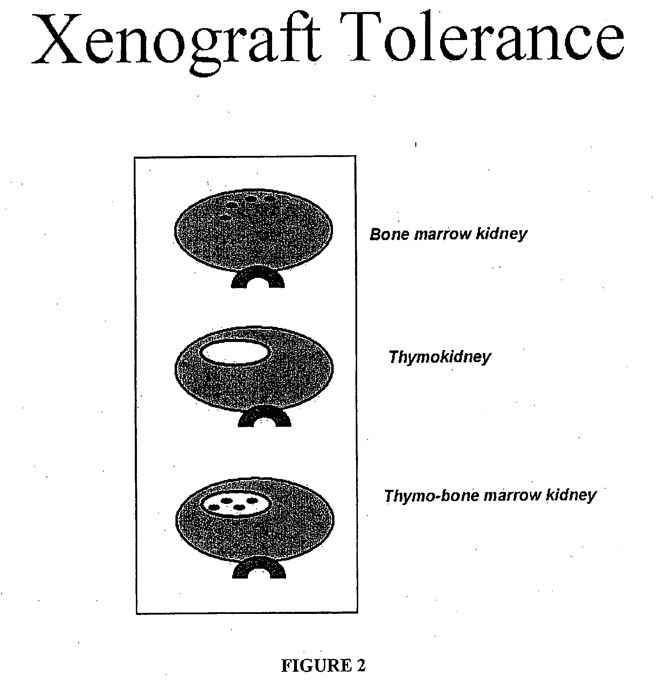

It is highly preferred that at least a portion of the thymic tissue component and the bone marrow tissue component be in direct contact. The surface area of direct contacting can be optimized by dividing the available thymic and bone marrow tissues into portions, and co-mingling a plurality of the combined tissue portions within the tissue composite construct or organ of the invention. Alternatively, the bone marrow plugs may be deposited onto the thymic graft tissue, as schematically depicted in Fig. 2.

In order to maintain structural integrity of the composite as well as to facilitate intimate association of the tissues, it is advantageous to surround the tissues with a biocompatible permeable or semipermeable membraneous material which is permissive of vascularization of the enclosed tissues. Such a material may conveniently be derived, from the intestinal sac of the tissue donor.

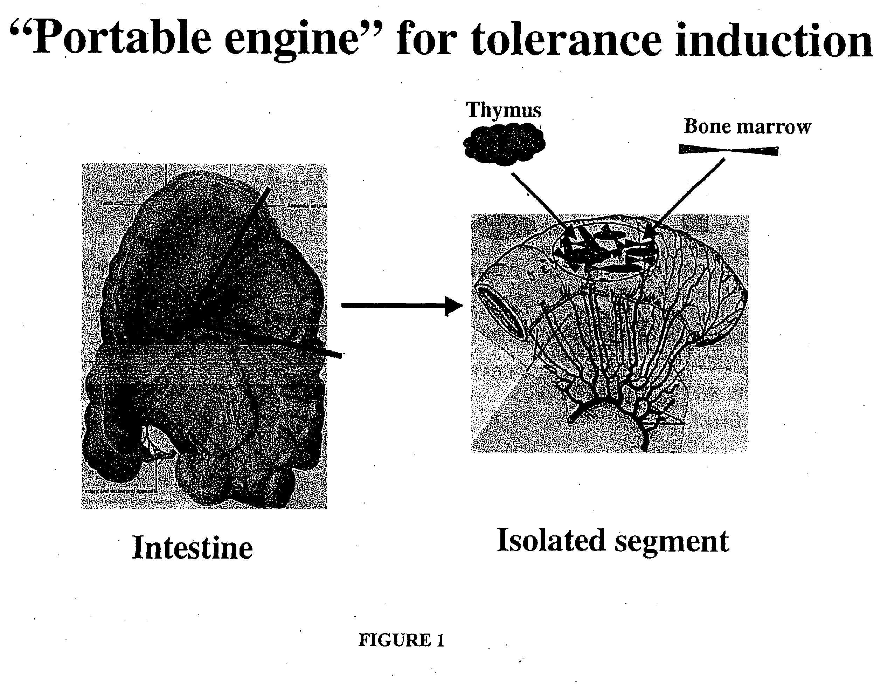

Thus one embodiment of the composite tissue construct of the invention comprises co-mingled sections or plugs of thymic tissue and HSM tissue of the bone marrow surrounded by a supporting means derived from intestinal sac. Such a 15 composite tissue construct is depicted in Figure 1.

Once prepared, this composite tissue construct in which the tissues coexist in mingled fashion, can then be surgically implanted in a vascularized transplantable organ such as a kidney or heart, or elsewhere in a pre-selected organ donor.

It will be expected that the composite tissue construct, when implanted into a mammal, will initially undergo at least a certain amount of atrophy and cell loss. However, in a blood rich host environment, the implanted tissue will rapidly become revascularized by the host (as depicted in Figure 1, right panel) so that the tissues resume their normal functioning.

Thus, it may be to administer conventional immunosuppressant treatment to the transplant patient necessary for a finite period of time following the transplantation for up to about +120 days, or preferably for up to about +90 days,.g., +30 13 days). Examples of suitable compounds include cyclosporins, rapamycins or ascomycins, or their immunosuppressive analogs, e.g. cyclosporin A, cyclosporin G, in FK-506, rapamycin, 40-0-(2-hydroxy)ethyl-rapamycin; corticosteroids; cyclophosphamide; azathioprene; methotrexate; brequinar; leflunomide; mizoribine; mycophenolic acid; mycophenolate mofetil (MMF); deoxysperguahns (e.g., 15 deoxysperguahne) and analogs, 2-amino-2-[2-(4-octylphenyl)ethyllpropane-1,3-diol hydrochloride, corticosteroids (e.g., methotrexate, prednisolone, methylprednisolone, dexamethasone), or other immunomodulatory compounds (e.g., CTLA4-1g); antiLFA- I or anti-ICAM antibodies, or antibodies to leukocyte receptors or their ligands 10 (e.g. antibodies to MHC, CD2, CD3, CD4, CD7, CD25, CD28, B7, CD40, CD45, CD58, CD152 (CTLA-4), or CD 154 (CD40 figand).

The composite tissue construct of the invention comprising thymic and HSM tissue of the bone marrow can be surgically implanted directly into a pre- selected organ of a donor animal a pig) for eventual transplantation into a recipient mammal (e.g., human). Alternatively, but less preferably, rather than first preparing a composite tissue construct, it is possible to implant each of the thymic tissue component and the HSM bone marrow component as a discrete component in the organ to form a composite organ of the invention.

The resulting organ containing the grafted tissues in intimate association, and preferably in a contacting relationship, is referred to as a "thymo-bone marrow" (or "thy-marrow") composite organ. The pre- selected organ may comprise, for example, kidney, heart, lung, combined heart-lung, liver, or pancreas. An embodiment of a thymo-bone marrow ('thy-marrow')-kidney is schematically depicted in Fig. 2.

The implanting of the thymic and bone marrow tissues may be carried out in vivo (i.e. while the organ is still in situ in the living donor animal) or ex vivo (i.e. after the organ has been removed from the donor and before it is transplanted to the recipient) or even in vivo in the transplant recipient at the same time as the organ blood vessels are being anastemosed to the corresponding blood vessels of the recipient.

In another embodiment of the invention, the discrete thymic and HSM-bone marrow tissues or composite tissue construct may be implanted into the intended organ donor, not initially in the organ to be transplanted, but at another suitable blood suffused location. The composite tissue construct can then be transferred to the selected organ at a later time including up to the time of transplantation.

In a further, preferred embodiment of the invention, the composite organ may be maintained in vivo in the donor animal, before being harvested for transplantation, for a sufficient time and under conditions allowing the implanted tissues to become vascularized in the donor. A suitable period of residency to permit vascularization of a thymo-bone marrow-kidney, for example, may be from about one to about six months.

In this case, prior to the actual organ transplant event, it is desirable that the composite organ be pre-conditioned by known methods by T-cell depletion or irradiation, whether in vivo or ex vivo (i.e. after being removed from the donor but prior to transplantation into the recipient).

Optionally, pre-conditioning of the recipient to deplete mature T cells may precede transplantation of the tissue composite construct or the thymo-bone marrow composite organ of the invention. Preconditioning may, for example, be carried out by irradiation or by administering to the patient sufficient immunotoxin such as disclosed in PCT WO 98/39363 to deplete the patient's T-cell population by, e.g., at least 2, and preferably at least 3, logs. Recipients of the thy-marrow composite tissue constructs and organs of the invention are mixed hematopoietic chimeras possessing bone marrow precursor cells of both recipient and donor origin. Both types of mature T cell populations are present in the recipient, the donor T-cells being restricted through positive selection in the 15 thymus to the recognition of donor MHC + antigen. Host antigen-presenting cells (APQ in the periphery ensure that in-immunocompetent interactions can occur. In addition, such recipients receive a self-replenishing source of donor APC from the implanted hematopoietic stromal microenvironment of the donor (or donor histocompatible) bone marrow, including dendritic cells which localize in the corticomedullary junction of the implanted thymic tissue. As donor thymocytes differentiate and mature in the implanted thymic tissue, they pass through this site, and are subjected to negative selection to the donor in the same way that host thymocytes were negatively selected for self in the host thymus, rendering the transplanted individual both immunocompetent and tolerant to self-as well as to donor. In this way, the thy-marrow tissue composite of the invention provides a "portable engine" for inducing tolerance in the transplanted individual.

The "portable engine" of the invention is particularly useful in establishing hematopoietic chimerism and inducing tolerance in xenotransplant recipients, and in particular in human recipients of pig organs.

The following examples are for illustrative purposes only and are not to be construed as limitative of the scope of the invention in any manner.

### Examples

### Bone marrow/thymus implantation under the renal capsule

Donor and recipient mice of C57BU6 strain obtained from Jackson Labs are anesthetized with cocktail of Ketamine (100 mg/kg) and xylazine (4 mg/kg). All of the long bones of the donor animal are removed, and hematopoietic stromal tissue is collected by removing the neck of the bone and flushing the marrow cavity with cold isotonic saline. The collected bone marrow is pooled and pelleted into a tube.

Donor thymus is collected and transferred to a sterile tissue culture dish filled with sterile cold saline. The thymus is divided into small piece with scissors (2 mm x 4 MM).

### A. Bone marrow implantation:

The recipient animal is placed on its right side. The left kidney is exposed via an incision of 10 to 12 mm. A 1 mm. cut is made with a fine tip forceps through the kidney capsule for tissue implanting, creating a tunnel). Syngeneic bone marrow tissue is delivered under the renal capsule (Fig-2 upper panel and Fig-3 left panel). Upon completion of implantation, the abdomen incision is sutured closed.

The implanted bone marrow is harvested for histology assessment at various intervals. Extensive angiogensis forms around bone marrow implanting site (Fig-3 right panel). At 5 weeks post-transplantation, histology evaluation shows "regenerated" bone marrow under kidney capsule (Fig-4 right panel).

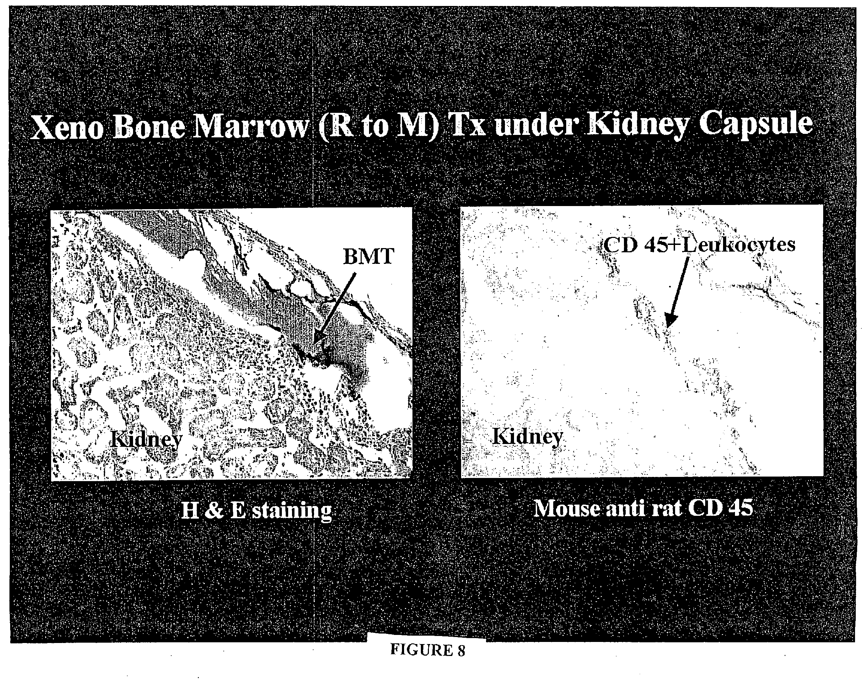

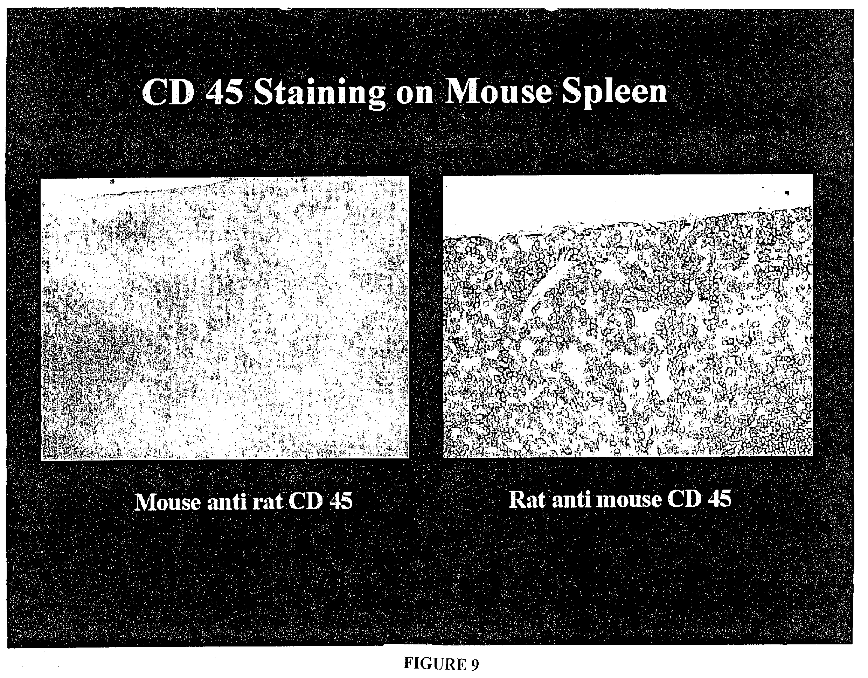

This regenerated bone marrow tissue is also observed in a xenogeneic setting, i.e. rat to mouse, either at early (Fig-5) or later time points (Fig-6). Immunohistochemistry reveals that the cells present under the renal capsule are donor (i.e. rat)origin CD45+ leukocytes (Fig. 8, Fig. 9).

### B. Bone marrow/thymus implantation

The preparation of the recipient animal is as described above. Bone marrow tissue is delivered under the renal capsule followed by thymus tissue (Fig-2 lower panel). This procedure is repeated several times. The final size of the implant is about 6 mm x 4 mm. Histology evaluation at 4 weeks post-transplantation shows regenerated bone marrow and thymus components under the renal capsule (Fig-7).

## Claims

1. A composite tissue construct for implantation into a mammal comprising:
(a) a vascularizable first tissue component comprising functional thymic tissue;
(b) a vascularizable second tissue component, histocompatible to the first tissue component, which comprises a functional hematopoietic stromal microenvironment (HSM) of the bone marrow, and
(c) supporting means for maintaining said first and second tissue components in intimate association.

2. A composite tissue construct according to claim 1 wherein at least a portion of the functional thymic tissue of the first tissue component and at least a portion of the functional FISM of the bone marrow of the second tissue component are in a contacting relationship.

3. A composite tissue construct according to claim 1 wherein the thymic tissue of the first component and the HSM of the bone marrow of the second tissue component are obtained from swine.

4. A composite tissue construct according to claim 1 wherein the supporting means comprises a segment of mammalian intestinal sac.

5. A composite organ for transplantation comprising:
(a) a mammalian organ having means for being joined to the blood circulation of a mammal,
(b) a vascularizable first tissue component comprising functional thymic tissue, and
(c) a vascularizable second tissue component in intimate association with said second tissue component, comprising a functional hematopoietic stromal microenvironment (HSM) of the bone marrow, said first and second tissue components being histocompatible to the mammalian organ.

6. A composite organ according to claim 5 wherein at least a portion of the functional thymic tissue of the first tissue component and at least a portion of the functional HSM of the bone marrow of the second tissue component are in a contacting relationship.

7. A composite organ according to claim 5 wherein the first tissue component and the second tissue component are contained by a biocompatible supporting means permissive of vascularization of said first and second tissue components.

8. A composite organ according to claim 5 which is a kidney.

9. A composite organ according to claim 5 which is a heart.

10. A composite organ according to claim 5 wherein the mammalian organ is of a swine.

11. A composite organ according to claim 5 wherein the first and second tissue components are of autologous or non-autologous swine origin.

12. A non-human mammal comprising a composite organ according to claim 5.

13. A non-human mammal comprising a composite organ according to claim 1 which is a pig.
